(19) Europäisches Patentamt
European Patent Office
Office européen des brevets

(11) **EP 4 296 309 A1**

(12) **EUROPEAN PATENT APPLICATION**
published in accordance with Art. 153(4) EPC

(43) Date of publication:
**27.12.2023 Bulletin 2023/52**

(21) Application number: **22756143.8**

(22) Date of filing: **15.02.2022**

(51) International Patent Classification (IPC):
**C08L 1/10** (2006.01)  **A61K 8/02** (2006.01)
**A61K 8/19** (2006.01)  **A61K 8/73** (2006.01)
**C08K 3/22** (2006.01)  **C08K 3/26** (2006.01)
**C08K 5/10** (2006.01)

(52) Cooperative Patent Classification (CPC):
**A61K 8/02; A61K 8/19; A61K 8/73; C08K 3/22;
C08K 3/26; C08K 5/10; C08L 1/10**

(86) International application number:
**PCT/JP2022/005803**

(87) International publication number:
**WO 2022/176825 (25.08.2022 Gazette 2022/34)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **19.02.2021 JP 2021024893**

(71) Applicant: **Daicel Corporation
Osaka 530-0011 (JP)**

(72) Inventors:
• **OMURA, Masaya**
  **Tokyo 108-8230 (JP)**
• **KOBAYASHI, Keiko**
  **Tokyo 108-8230 (JP)**
• **MAKINO, Junko**
  **Tokyo 108-8230 (JP)**

(74) Representative: **Grünecker Patent- und
Rechtsanwälte
PartG mbB
Leopoldstraße 4
80802 München (DE)**

(54) **CELLULOSE ACYLATE COMPOSITION AND PRODUCTION METHOD THEREFOR**

(57)  Provided is a cellulose acylate composition containing cellulose acylate particles and metal compound-containing particles. The total degree of substitution of the cellulose acylate is from 0.7 to 2.9. The metal compound is one or more types of compounds selected from an alkali metal compound and an alkaline earth metal compound. The composition is produced by adding one or more types of metal compounds selected from an alkali metal compound and an alkaline earth metal compound during and/or after removing a water-soluble polymer from a dispersion containing, as a dispersoid, cellulose acylate impregnated with a plasticizer to form cellulose acylate particles.

FIG. 1

Processed by Luminess, 75001 PARIS (FR)

EP 4 296 309 A1

## Description

Technical Field

The present disclosure relates to a cellulose acylate composition and a method for producing the same. Specifically, the present disclosure relates to a cellulose acylate composition for use in a cosmetic composition, and to a method for producing the cellulose acylate composition.

Background Art

Typically, various fine polymer particles are blended in cosmetics for purposes such as improving the spread of the cosmetic, changing the tactile sensation, imparting a wrinkle blurring effect, and improving the lubricity of a foundation or the like. In particular, fine particles with high sphericity are excellent in imparting good tactile sensation and, depending on the physical properties and shape of the fine particles, can provide a light-scattering (soft-focus) effect. When used in a foundation or the like, such fine particles fill and smooth out the roughness of the skin, and scatter light in various directions, and thus can be expected to have an effect of making wrinkles and the like less noticeable (soft-focus effect).

Examples of such fine particles that are blended into cosmetic products include fine particles including a synthetic polymer, such as polyamide, polymethyl methacrylate (PMMA), polystyrene, polypropylene, and polyethylene. However, in recent years, application of fine particles including cellulose, which is a natural polymer, or of a cellulose derivative, which is a semi-synthetic polymer, in place of these synthetic polymers has been examined from the viewpoint of the environmental concern.

Patent Document 1 describes a method including: forming a polysaccharide ester product from a polysaccharide synthesis, in which the polysaccharide ester product contains a polysaccharide ester and a solvent; diluting the polysaccharide ester product and thereby yielding a polysaccharide ester dope; and forming a plurality of polysaccharide ester microspheres from the polysaccharide ester dope; and lists a cosmetic composition as an article that can contain a polysaccharide ester microsphere.

Patent Document 2 describes a cellulose acylate having a volume average particle size D50 from 72 $\mu$m to 100 $\mu$m, as measured using a laser diffraction particle size distribution measuring apparatus, a degree of polymerization from 131 to 350, and a degree of substitution from 2.1 to 2.6. Patent Document 2 also indicates that a preferable method for producing the cellulose acylate includes: acylating cellulose in the presence of sulfuric acid; and deacylating the acylated cellulose in a polar solvent in the presence of acetic acid.

Patent Document 3 describes producing a molded article, including kneading a resin component (A), such as a thermoplastic resin, and a water-soluble auxiliary component (B) to prepare a dispersion; and eluting the auxiliary component (B) from the dispersion to produce a molded article (e.g., a porous article or spherical particles) constituted of the resin component (A); and also describes a cellulose derivative, such as cellulose acetate, as the resin component (A).

Patent Document 4 describes cellulose acetate particles having an average particle size from 80 nm to 100 $\mu$m, a sphericity from 0.7 to 1.0, a degree of surface smoothness from 80% to 100%, and a total degree of acetyl substitution from 0.7 to 2.9.

Citation List

Patent Documents

    Patent Document 1: JP 2016-500129 T

    Patent Document 2: JP 6187653 B

    Patent Document 3: JP 2004-051942 A

    Patent Document 4: JP 6609726 B

Summary of Invention

Technical Problem

However, the polysaccharide ester microspheres of Patent Document 1 are porous particles having a large particle size and a broad particle size distribution and thus are not sufficient as an alternative to fine particles of a synthetic polymer to be included in cosmetics or the like. In addition, the cellulose acylates obtained by the production method described in Patent Document 2 are also porous particles of irregular shapes. Furthermore, the particulate molded article obtained by the production method described in Patent Document 3 also has a low sphericity and is particles that are approximately spherical.

The cellulose acetate particles disclosed in Patent Document 4 excel in good tactile sensation due to the high sphericity and surface smoothness, and when the cellulose acetate particles are blended in a cosmetic composition, the cosmetic composition can exhibit a light scattering (soft focus) effect. In the field of cosmetic compositions, there is a strong demand for fine particles conforming to the Japanese Standards of Quasi-Drug Ingredients. In addition, a demand exists for fine particles that do not change over time even during long-term storage and that can be blended into cosmetic compositions while conforming to these standards (in particular, the pH standard). Moreover, there is a room for further improvements in the stability over time of typically known cellulose acetate particles.

Thus, an object of the present disclosure is to provide a cellulose acylate composition that exhibits improved stability over time and maintains the excellent physical properties present at the initial stage of production for a long period of time. Another object of the present disclosure is to provide a method for producing the same.

Solution to Problem

As a result of intensive studies, the present inventors discovered that acetic acid or the like bonded to cellulose acylate is detached as a free acid over time, and as a result, the pH of the cellulose acylate composition deviates from the pH (6.0 to 8.0) stipulated by the Japanese Standards of Quasi-Drug Ingredients and the detached acetic acid or the like can become an odor-causing factor.

The cellulose acylate composition according to the present disclosure includes cellulose acylate particles and metal compound-containing particles, and the total degree of substitution of the cellulose acylate is from 0.7 to 2.9. The metal compound is one or more types of compounds selected from an alkali metal compound and an alkaline earth metal compound.

Preferable metal compounds are hydroxides, oxides, or carbonates of alkali metals or alkaline earth metals. The preferred alkali metal is sodium. The preferred alkaline earth metal is calcium or magnesium.

The metal compound-containing particles preferably have an average particle size from 20 nm to 20 μm. Furthermore, the metal compound-containing particles are preferably aggregates.

The cellulose acylate composition preferably contains the alkali metal and the alkaline earth metal at a total concentration from 50 ppm to 2000 ppm.

The average particle size of the cellulose acylate particles is preferably from 80 nm to 100 μm. Moreover, the cellulose acylate particles preferably have a sphericity from 0.7 to 1.0. Further, the degree of surface smoothness of the cellulose acylate particles is preferably from 80% to 100%.

Preferably, a substituent of the cellulose acylate is an acyl group having from 2 to 20 carbons. The acyl group is preferably selected from an acetyl group, a propionyl group, and a butyryl group.

The cellulose acylate particles preferably contain a plasticizer. The content of the plasticizer is from 2 wt.% to 40 wt.% relative to the cellulose acylate particles. A preferable plasticizer is one or more types selected from the group consisting of a citrate-based plasticizer, a glycerin ester-based plasticizer, an adipate-based plasticizer, and a phthalate-based plasticizer.

A cosmetic composition according to an embodiment of the present disclosure includes any of the cellulose acylate compositions described above.

A method for producing a cellulose acylate composition according to an embodiment of the present disclosure includes:

(1) a first step of mixing cellulose acylate having a total degree of substitution from 0.7 to 2.9 and a plasticizer to form a cellulose acylate impregnated with the plasticizer;
(2) a second step of kneading a water-soluble polymer and the cellulose acylate impregnated with the plasticizer at a temperature from 200°C to 280°C to form a dispersion containing, as a dispersoid, the cellulose acylate impregnated with the plasticizer; and
(3) a third step of removing the water-soluble polymer from the dispersion to form cellulose acylate particles. In this production method, one or more metal compounds selected from an alkali metal compound and an alkaline earth metal compound are added to the cellulose acylate particles during and/or after the third step.

Preferably, during the third step, the metal compound is added to the cellulose acylate particles by washing the dispersion at least once with a solution containing the metal compound. Preferably, the solution containing the metal compound is an aqueous solution.

Preferably, the amount of the metal compound added per 100 parts by weight of the cellulose acylate particles is from 0.01 parts by weight to 1.0 parts by weight.

Advantageous Effects of Invention

The cellulose acylate composition according to an embodiment of the present disclosure contains alkali metal compound-containing particles and/or alkaline earth metal compound-containing particles. These metal compound-containing particles suppress the detachment of free acid from the cellulose acylate. According to this cellulose acylate composition, a change over time caused by detachment a free acid can be avoided. The cellulose acylate composition exhibits excellent stability over time. A cosmetic composition containing the cellulose acylate composition can maintain the excellent physical properties present at the initial stage of production even after the passage of a long period of time.

Brief Description of Drawings

FIG. 1 is an electron micrograph of a cellulose acylate composition of Example 1.
FIG. 2 is an electron micrograph of Comparative Example 1.

Description of Embodiments

Hereinafter, an example of a preferred embodiment will be specifically described. Note that the configurations, combinations thereof, and the like in the embodiments are each an example, and various additions, omissions, substitutions, and other changes may be made as appropriate without departing from the spirit of the present disclosure. The present disclosure is not limited by the embodiments and is limited only by the claims. Each aspect disclosed in the present specification can be combined with any other feature disclosed herein.

In the present specification, "from X to Y" indicating a range means "X or greater and Y or less". Unless otherwise noted, all test temperatures are room temperature (20°C ± 5°C).

[Cellulose Acylate Composition]

The cellulose acylate composition according to an embodiment of the present disclosure includes cellulose acylate particles and metal compound-containing particles. The total degree of substitution of the cellulose acylate is from 0.7 to 2.9. The metal compound is one or more types of compounds selected from an alkali metal compound and an alkaline earth metal compound. The cellulose acylate composition may contain other metal compounds as inevitable impurities.

[Metal Compound-Containing Particles]

In the cellulose acylate composition according to an embodiment of the present disclosure, a main component of the metal compound-containing particles is a metal compound selected from an alkali metal compound and an alkaline earth metal compound. The metal compound-containing particles function as a neutralizing agent for the cellulose acylate particles. The metal compound-containing particles contribute to suppression of the detachment of acetic acid or the like bonded to the cellulose acylate. With the cellulose acylate composition, a decrease in pH due to the generation of a free acid such as acetic acid is avoided, and therefore, for example, even during long-term storage, the pH is maintained within the range defined in the Japanese Standards of Quasi-Drug Ingredients, and generation of an odor due to acetic acid or the like is also suppressed. Further, when the cellulose acylate composition includes the metal compound-containing particles, the stability of the cellulose acylate composition over time is improved.

In addition, in the cellulose acylate composition according to an embodiment of the present disclosure, the metal compound-containing particles are present substantially independent from the cellulose acylate particles, and differ from a metal compound originating from an additive, an impurity, or the like during the production of the cellulose acylate serving as a raw material. According to the findings of the present inventors, the metal compound-containing particles function as an antiblocking agent and suppress aggregation of the cellulose acylate particles. As a result, with the cellulose acylate composition according to an embodiment of the present disclosure, in addition to an improvement in tactile sensations such as smoothness and a moist feeling, a tactile sensation is further improved in the sense that the composition is less likely to form lumps. When the cellulose acylate composition according to an embodiment of the present disclosure is blended into a cosmetic composition, the cellulose acylate composition can maintain the excellent quality at the time of production at a high level for a long period of time.

Examples of the metal compound include a compound of an alkali metal such as sodium, lithium, and potassium, and a compound of an alkaline earth metal such as calcium, magnesium, and barium. Sodium is preferred as the alkali metal, and calcium and magnesium are preferred as the alkaline earth metal. Examples thereof include a hydroxide such as sodium hydroxide, calcium hydroxide, and magnesium hydroxide; an oxide such as sodium oxide, calcium oxide, and magnesium oxide; and a carbonate such as sodium carbonate, calcium carbonate, and magnesium carbonate. Calcium carbonate, calcium hydroxide, magnesium carbonate, and magnesium hydroxide are more preferable. For example, when the metal compound is calcium hydroxide, calcium carbonate may be produced in some cases by a reaction with carbon dioxide in the air, and such a cellulose acylate composition is also included in the technical scope of the present application.

The average particle size of the metal compound-containing particles is preferably from 20 nm to 20 $\mu$m. From the viewpoint of ease of production, the average particle size of the metal compound-containing particles is more preferably 50 nm or larger, and even more preferably 1 $\mu$m or larger. From the viewpoint of improving the tactile sensation, the average particle size of the metal compound-containing particles is more preferably 15 $\mu$m or smaller, and even more preferably 12 $\mu$m or smaller. The average particle size of the metal compound-containing particles is determined by measuring the major axis length and the minor axis length of each particle using an image of the particles observed with a scanning electron microscope (SEM), calculating the average values thereof, and obtaining a number average value of 30 particles selected at random.

In the cellulose acylate composition according to an embodiment of the present disclosure, the metal compound-containing particles may be aggregates. When the metal compound-containing particles are aggregates, the average particle size of the aggregates is preferably within the numerical range described above.

The content of the metal compound-containing particles in the cellulose acylate composition according to an embodiment of the present disclosure is not particularly limited, but the cellulose acylate composition preferably contains the alkali metal and the alkaline earth metal at a total concentration from 50 ppm to 2000 ppm. When the cellulose acylate composition contains the alkali metals and the alkaline earth metals at a total amount of 50 ppm or greater, a decrease in pH is effectively suppressed, and the stability over time is improved. When the total concentration of the alkali metal and the alkaline earth metal exceeds 2000 ppm, the pH value may increase and deviate from the desired range during production. The concentration of metal in the cellulose acylate composition is measured by absorption photometry using an atomic absorption photometer. Details will be described later in the Examples.

[Cellulose Acylate Particles]

The average particle size of the cellulose acylate particles may be from 80 nm to 100 $\mu$m, 100 nm or greater, 1 $\mu$m or greater, 2 $\mu$m or greater, or 4 $\mu$m or greater. In addition, the average particle size may be 80 $\mu$m or less, 40 $\mu$m or less, 20 $\mu$m or less, or 14 $\mu$m or less. The particles with too large average particle size may have poor tactile sensation and a reduced light-scattering (soft-focus) effect. Alternatively, the particles with too small average particle size may be difficult to produce. Here, the tactile sensation includes a tactile sensation resulting from direct contact with the cellulose acylate particles, and also includes, for example, the skin feel and tactile sensation resulting from use of a cosmetic composition containing the particles.

The average particle size can be measured using dynamic light scattering. The average particle size is measured specifically as follows. First, a sample is prepared by adding the cellulose acylate particles to pure water to make a concentration of 100 ppm, and forming a pure water suspension using an ultrasonic vibrating device. Subsequently, the volume-frequency particle size distribution is measured by laser diffraction (using the "Laser Diffraction/Scattering Particle Size Distribution Measuring Device LA-960" available from Horiba Ltd., ultrasonic treatment for 15 minutes, refractive index (1.500, medium (water; 1.333)). The particle size corresponding to 50% of the integrated scattering intensity in the volume-frequency particle size distribution is determined as the average particle size. That is, the average particle size (nm or $\mu$m) in the present specification is a volume-based median diameter.

A coefficient of the particle size variation of the cellulose acylate particles may be from 0% to 60%, or may be from 2% to 50%. The coefficient of the particle size variation (%) can be calculated by an equation: (standard deviation of particle size)/(average particle size) x 100.

The sphericity of the cellulose acylate particles is preferably from 0.7 to 1.0, more preferably from 0.8 to 1.0, and even more preferably from 0.9 to 1.0. Cellulose acylate particles with a sphericity of less than 0.7 have poor tactile sensation, and, for example, a cosmetic composition containing such particles has a poor skin feeling and a reduced soft-focus effect.

The sphericity can be measured by the following method. From an image of particles observed with a scanning electron microscope (SEM), 30 particles are randomly selected, the major axis length and the minor axis length of the 30 randomly selected particles are measured, the (minor axis length)/(major axis length) ratio of each particle is determined, and then the average value of the (minor axis length)/(major axis length) ratios is defined as the sphericity. The particles can be determined to be closer to a true sphere as the sphericity approaches 1.

The degree of surface smoothness of the cellulose acylate particles is preferably from 80% to 100%, more preferably from 85% to 100%, and even more preferably from 90% to 100%. When the degree of surface smoothness is less than 80%, the tactile sensation may be poor. The tactile sensation becomes more preferable as the degree of surface smoothness becomes closer to 100%.

The degree of surface smoothness can be determined by capturing a scanning electron micrograph of the particles, observing recesses and protrusions of the particle surfaces, and determining the degree of surface smoothness on the basis of the area of recessed portions on the surfaces. Details will be described later in the Examples.

The total degree of substitution of the cellulose acylate constituting the cellulose acylate particles is from 0.7 to 2.9, is preferably 0.7 or greater and less than 2.6, is more preferably 1.0 or greater and less than 2.6, and is even more preferably 2.0 or greater and less than 2.6. A cellulose acylate having such a total degree of substitution is preferable because such a cellulose acylate excels in moldability and can easily produce spherical particles with high sphericity.

When the total degree of substitution is less than 0.7, water solubility increases, the cellulose acylate tends to elute in a step of extracting particles in the production of the cellulose acylate particles described below and particularly in a step of removing the water-soluble polymer from the dispersion. This may reduce the sphericity of the resulting particles and thus may lead to poor tactile sensation.

The total degree of substitution of the cellulose acylate can be measured by the following method. First, the total degree of substitution of the cellulose acylate is the sum of each degree of substitution at the 2-, 3-, and 6-positions of the glucose ring of the cellulose acylate, and each degree of substitution at the 2-, 3-, and 6-positions of the glucose ring of the cellulose acylate can be measured by NMR according to the Tezuka method (Tezuka, Carbonydr. Res. 273, 83 (1995)). That is, the free hydroxyl group of the cellulose acylate is acylated with a carboxylic anhydride in pyridine. The type of the carboxylic anhydride used here should be selected according to the purpose of the analysis; for example, when the degree of propionyl substitution of cellulose acetate propionate is analyzed, acetic anhydride is suitable, and when the degree of acetyl substitution is analyzed, propionic anhydride is suitable. The solvent and the acid anhydride of the acylation reaction may be appropriately selected according to the cellulose acylate to be analyzed.

A sample obtained by acylation is dissolved in deuterochloroform and the $^{13}$C-NMR spectrum is measured. For example, when the substituent is an acetyl group, a propionyl group, or a butyryl group, the carbon signals of the acetyl group appear in the region from 169 ppm to 171 ppm in the order of the 2-, 3-, and 6-positions from the high magnetic field; the carbonyl carbon signals of the propionyl group appear in the region from 172 ppm to 174 ppm in the same order; and the carbon signals of the butyryl group appear likewise in the region from 171 ppm to 173 ppm in the order of the 2-, 3-, and 6-positions from the high magnetic field side. In another example, when a cellulose acylate having a propionyl group is analyzed, or when a cellulose acylate having no propionyl group is treated with propionic anhydride and then the degree of propionyl substitution is analyzed, carbonyl carbon signals of the propionyl group appear in the same order in a region from 172 ppm to 174 ppm.

According to the Tezuka method or a method corresponding thereto, the total degree of substitution of the cellulose acylate treated with carboxylic anhydride is 3.0. Therefore, if the sum of the areas of the carbonyl carbon signal of the acyl group that originally belongs to the cellulose acylate and the carbonyl signal of the acyl group introduced by the carboxylic anhydride treatment is standardized at 3.0, and the presence ratio (the area ratio of each signal) of the acetyl group and the propionyl group at each corresponding positions is determined, each degree of acyl substitution at the 2-, 3-, and 6-positions of the glucose ring in the original cellulose acylate can be determined. It goes without saying, a substituent containing an acyl group that can be analyzed by this method is only a substituent group that does not correspond to the carboxylic anhydride used in the treatment for an analytical purpose.

However, if it is known in advance that the total degree of substitution of the 2-, 3-, and 6-positions of the glucose ring of the cellulose acylate of a sample is 3.0, and all the substituents thereof are limited to substituents such as an acetyl group and a propionyl group, the NMR spectrum can be measured by dissolving the sample directly in deuterochloroform without acylation. If all the substituents are an acetyl group and a propionyl group, as in the case including acylation, the carbon signals of the acetyl group appear in the region from 169 ppm to 171 ppm in the order of 2-, 3-, and 6-positions from the high magnetic field, and the carbon signals of the propionyl group appear in the region from 172 ppm to 174 ppm in the same order, and thus the degree of substitution, such as each degree of acetyl and propionyl substitution at the 2-, 3-, and 6-positions of the glucose ring in the cellulose acylate, can be determined from the presence ratio of the acetyl group and the propionyl group at each corresponding position (in other words, the area ratio of each signal).

The cellulose acylate forming the cellulose acylate particles according to an embodiment of the present disclosure preferably has an acyl group having from 2 to 20 carbons. The number of carbons of the acyl group may be 3 or more, and may be 4 or more. The number of carbons of the acyl group may be 18 or fewer, or 16 or fewer. The cellulose acylate may have two or more types of acyl groups having a different number of carbons.

Examples of the acyl group having from 2 to 20 carbons include an acetyl group, a propionyl group, a butyryl group, a pentanoyl (valeryl) group, a hexanoyl group, a heptanoyl group, an octanoyl group, a nonanoyl group, an undecanoyl group, a dodecanoyl group, a tridecanoyl group, a tetradecanoyl (myristoyl) group, a pentadecanoyl group, a hexadecanoyl group, a heptadecanoyl group, and an octadecanoyl (stearoyl) group. One or more acyl groups selected from an acetyl group, a propionyl group, and a butyryl group are preferable.

The cellulose acylate particles may contain or need not contain a plasticizer. In the present disclosure, the plasticizer refers to a compound that can increase the plasticity of the cellulose acylate. The plasticizer is not particularly limited, and examples thereof include an adipate-based plasticizer containing an adipate ester, such as dimethyl adipate, dibutyl adipate, diisostearyl adipate, diisodecyl adipate, diisononyl adipate, diisobutyl adipate, diisopropyl adipate, diethylhexyl adipate dioctyl adipate, dioctyldodecyl adipate, dicapryl adipate, and dihexyldecyl adipate; a citrate-based plasticizer containing a citrate ester, such as acetyl triethyl citrate, acetyl tributyl citrate, isodecyl citrate, isopropyl citrate, triethyl citrate, triethylhexyl citrate, and tributyl citrate; a glutarate-based plasticizer containing a glutarate ester, such as diisobutyl glutarate, dioctyl glutarate, and dimethyl glutarate; a succinate-based plasticizer containing a succinate ester, such as diisobutyl succinate, diethyl succinate, diethylhexyl succinate, and dioctyl succinate; a sebacate-based plasticizer containing a sebacate ester, such as diisoamyl sebacate, diisooctyl sebacate, diisopropyl sebacate, diethyl sebacate, diethylhexyl sebacate, and dioctyl sebacate; a glycerin ester-based plasticizer containing a glycerin alkyl ester, such as triacetin, diacetin, and monoacetin; neopentyl glycol; and a phosphate-based plasticizer containing a phosphate ester, such as trioleyl phosphate, tristearyl phosphate, and tricetyl phosphate. These plasticizers may be used alone, or two or more of the plasticizers may be used in combination.

Among these, preferable plasticizers are one or more selected from the group consisting of a citrate-based plasticizer containing a citrate ester, such as triethyl citrate, acetyl triethyl citrate, and acetyl tributyl citrate; a glycerin ester-based plasticizer containing a glycerin alkyl ester, such as triacetin, diacetin, and monoacetin; and an adipate-based plasticizer, such as diisononyl adipate; more preferable plasticizers are one or more selected from the group consisting of triethyl citrate, acetyl triethyl citrate, acetyl tributyl citrate, triacetin, and diisononyl adipate; and even more preferable plasticizers are one or more selected from the group consisting of acetyl triethyl citrate, triacetin, and diacetin. A phthalate-based plasticizer can be used, but it must be used with care because of concerns about similarity to environmental hormones.

When the cellulose acylate particles contain a plasticizer, the content of the plasticizer included in the cellulose acylate particles is not particularly limited. For example, in relation to the weight of the cellulose acylate particles, the content of the plasticizer may be from greater than 0 wt.% to 40 wt.%, from 2 wt.% to 40 wt.%, from 10 wt.% to 30 wt.%, or from 15 wt.% to 20 wt.%.

The content of the plasticizer in the cellulose acylate particles is determined by dissolving the cellulose acylate particles in a solvent that can dissolve the cellulose acylate particles, and then measuring the obtained solution by $^1$H-NMR.

The cellulose acylate composition according to an embodiment of the present disclosure can be produced by a production method described below.

The cellulose acylate composition according to an embodiment of the present disclosure exhibits good stability over time and excellent tactile sensation, and thus can be suitably used in a cosmetic composition. In addition, the cellulose acylate particles have high sphericity, and thus when blended into a cosmetic composition, the cellulose acylate particles fill in and smooth out the recesses and protrusions of the skin and scatter light in various directions, and thus provide an effect of making wrinkles and the like less noticeable (soft-focus effect). Furthermore, the cellulose acylate composition according to an embodiment of the present disclosure can maintain such excellent physical properties present immediately after production for a long period of time.

Examples of the cosmetic composition include foundations, such as liquid foundations and powder foundations; concealers; sunscreens; makeup bases; lipsticks and lipstick bases; Oshiroi face powders, such as body powders, solid white powders, and face powders; solid powder eye shadows; wrinkle masking creams; and skin and hair external preparations used mainly for cosmetic purposes, such as skin care lotions; and the dosage form is not limited. The dosage form may be any of a liquid preparation, such as an aqueous solution, a milky lotion, and a suspension; a semisolid preparation, such as a gel and a cream; or a solid preparation, such as a powder, a granule, and a solid. In addition, the dosage form may be an emulsion preparation, such as a cream and a milky lotion; an oil gel preparation, such as a lipstick; a powder preparation, such as a foundation; an aerosol preparation, such as a hair styling agent; or the like.

[Method for Producing Cellulose Acylate Composition]

A method for producing a cellulose acylate composition according to an embodiment of the present disclosure includes: a first step of mixing cellulose acylate having a total degree of substitution from 0.7 to 2.9 and a plasticizer to form a cellulose acylate impregnated with the plasticizer; a second step of kneading a water-soluble polymer and the cellulose acylate impregnated with the plasticizer at a temperature from 200°C to 280°C to form a dispersion containing, as a dispersoid, the cellulose acylate impregnated with the plasticizer; and a third step of removing the water-soluble polymer from the dispersion to form cellulose acylate particles. One or more metal compounds selected from an alkali metal compound and an alkaline earth metal compound are added to the cellulose acylate particles during and/or after the third step, and thereby the cellulose acylate composition according to an embodiment of the present disclosure is produced.

(First Step)

In the first step of forming cellulose acylate impregnated with a plasticizer, cellulose acylate having a total degree of substitution from 0.7 to 2.9 and a plasticizer are mixed.

The cellulose acylate having a total degree of substitution from 0.7 to 2.9 can be produced by a known production method. For example, the cellulose acylate can be produced by activating a raw material pulp (cellulose); acylating the activated cellulose using an esterifying agent (acylating agent); deactivating the acylating agent after the completion of the acylation reaction; and aging (saponifying, hydrolyzing) the produced cellulose acylate. In addition, the method may include, prior to the activation, pretreating the raw material pulp, including disintegrating and grinding the pulp, and then spraying and mixing acetic acid therewith. The method may include post-treating the resulting cellulose acylate, including precipitating and separating, purifying, stabilizing, and drying the cellulose acylate after the aging (saponifying, hydrolyzing).

The total degree of substitution of the cellulose acylate can be adjusted by adjusting the conditions of aging (conditions such as the time and temperature). The type of substituent is determined by selection of the esterifying agent. The total degree of substitution of the obtained cellulose acylate is from 0.7 to 2.9, preferably 0.7 or greater and less than 2.6, more preferably 1.0 or greater and less than 2.6, even more preferably 1.4 or greater and less than 2.6, and particularly preferably 2.0 or greater and less than 2.6.

The plasticizer is not particularly limited, and any plasticizer having a plasticizing effect in a process of melt-extruding cellulose acylate can be used. Specifically, the plasticizers described above as the plasticizer that is included in the cellulose acylate particles may be used alone, or two or more types of the plasticizers may be used in combination. One or more plasticizers selected from the group consisting of a citrate-based plasticizer, a glycerin ester-based plasticizer, an adipate-based plasticizer, and a phthalate-based plasticizer are preferable, and one or more plasticizers selected from the group consisting of acetyl triethyl citrate, triacetin, diacetin, and diethyl phthalate are more preferable.

The blended amount of the plasticizer per 100 parts by weight of the total amount of the cellulose acylate and the plasticizer may be greater than 0 parts by weight and 40 parts by weight and less, from 2 parts by weight to 40 parts by weight, from 10 parts by weight to 30 parts by weight, or from 15 parts by weight to 20 parts by weight. When the blending amount of the plasticizer is too small, the sphericity of the cellulose acylate particles tends to decrease, and when the blending amount is too large, the shape of the particles cannot be maintained, and the sphericity tends to decrease.

The cellulose acylate and the plasticizer can be dry-mixed or wet-mixed using a mixer, such as a Henschel mixer. When a mixer such as a Henschel mixer is used, the temperature in the mixer may be a temperature at which the cellulose acylate does not melt, for example, a temperature in a range of 20°C or higher and lower than 200°C.

In addition, the cellulose acylate and the plasticizer may be mixed by melt-kneading. Furthermore, the melt-kneading may be performed in combination with mixing with a mixer such as a Henschel mixer, and in this case, the melt-kneading is preferably performed after mixing at a temperature in a range of 20°C or higher and lower than 200°C using a mixer such as a Henschel mixer. Through such a method, the plasticizer and the cellulose acylate are more uniformly mixed well in a short period of time, and thus the sphericity of the obtained cellulose acylate particles is increased, and the tactile sensation and touch feeling are improved.

The melt-kneading can be carried out by heating and mixing with an extruder. The kneading temperature (cylinder temperature) of the extruder may be from 200°C to 230°C. The melt-kneading performed at a temperature in this range can plasticize the cellulose acylate and provide a uniform kneaded product. The melt-kneading performed at too low temperatures may reduce the sphericity of the resulting particles, thus reducing the tactile sensation and the touch feeling. The melt-kneading performed at too high temperatures may cause deterioration or coloration of the kneaded product due to heat Moreover, the viscosity of the melted material may decrease, and kneading of the resin in the extruder may be insufficient.

The melting point of the cellulose acylate depends on the type of substituent and the degree of substitution, but is approximately from 230°C to 280°C and is close to the decomposition temperature of the cellulose acylate. Thus, melt-kneading in this temperature range is typically difficult, but with the cellulose acylate (flakes) impregnated with the plasticizer, the plasticizing temperature can be reduced. In a case in which a twin-screw extruder is used, for example, the kneading temperature (cylinder temperature) may be 200°C. The kneaded product may be extruded in a strand shape and formed into a pellet form by hot cutting or the like. The die temperature in this case may be approximately 220°C.

(Second Step)

In the second step, a water-soluble polymer and the cellulose acylate impregnated with the plasticizer are kneaded at a temperature from 200°C to 280°C, and a dispersion containing the cellulose acylate as a dispersoid is obtained.

The water-soluble polymer and the cellulose acylate impregnated with the plasticizer can be kneaded with an extruder, such as a twin-screw extruder. The temperature of the kneading refers to the cylinder temperature.

The dispersion may be extruded in a string shape from a die attached to the tip of an extruder, such as a twin-screw extruder, and then cut into pellets. At this time, the die temperature may be from 220°C to 300°C.

The blended amount of the water-soluble polymer may be from 55 parts by weight to 99 parts by weight per 100 parts by weight of the total amount of the water-soluble polymer and the cellulose acylate impregnated with the plasticizer. The content is preferably from 60 parts by weight to 90 parts by weight, and even more preferably from 65 parts by weight to 85 parts by weight.

In the present specification, the water-soluble polymer refers to a polymer having an insoluble content of less than 50 wt.% when 1 g of the polymer is dissolved in 100 g of water at 25°C. Examples of such a water-soluble polymer may include polyvinyl alcohol, polyethylene glycol, sodium polyacrylate, polyvinylpyrrolidone, polypropylene oxide, polyglycerin, polyethylene oxide, vinyl acetate, modified starch, thermoplastic starch, methyl cellulose, ethyl cellulose, hydroxyethyl cellulose, and hydroxypropyl cellulose. Among these, polyvinyl alcohol, polyethylene glycol, and thermoplastic starch are preferred, and polyvinyl alcohol and thermoplastic starch are particularly preferred. Further, the thermoplastic starch can be prepared by a well-known method. For example, reference can be made to JP H06-06307 B, WO 92/04408, etc., and more specifically, for example, a thermoplastic starch prepared by mixing approximately 20% of glycerin as a plasticizer to tapioca starch and kneading them with a twin-screw extruder can be used.

The dispersion obtained in the second step is a dispersion containing the water-soluble polymer as a dispersion medium and the cellulose acylate impregnated with the plasticizer as a dispersoid. In other words, the dispersion may be a constitution containing the water-soluble polymer as a sea component and the cellulose acylate impregnated with the plasticizer as an island component. In the dispersion, the kneaded product constituting the island component contains the cellulose acylate and the plasticizer and is mainly spherical.

(Third Step)

In the third step of forming cellulose acylate particles, the water-soluble polymer is removed from the dispersion described above.

The method of removing the water-soluble polymer is not particularly limited as long as the water-soluble polymer dissolves and can be removed from the particles. Examples of such method include a method of dissolving and removing the water-soluble polymer in the dispersion using a solvent, such as water; an alcohol, such as methanol, ethanol, or isopropanol; or a mixed solution of water and such an alcohol. Specific examples include a method of removing the water-soluble polymer from the dispersion by mixing the dispersion and the solvent and then separating the solvent in which the water-soluble polymer is dissolved through a technique such as water-liquid separation through filtration or the like. When the water-soluble polymer is removed by a method of stirring and mixing the dispersion and the solvent and then filtering the mixture, the stirring, mixing, and filtration may be repeated a plurality of times for the purpose of increasing the removal efficiency.

In the third step, the plasticizer may or may not be removed from the dispersion together with the water-soluble polymer. Thus, the resulting cellulose acylate particles may or may not contain the plasticizer.

A mixing ratio of the dispersion and the solvent may be set such that in relation to the total weight of the dispersion and the solvent, the content of the dispersion is from 0.01 wt.% to 20 wt.%, from 2 wt.% to 15 wt.%, or from 4 wt.% to 13 wt.%. When the content of the dispersion is greater than 20 wt.%, the water-soluble polymer may not be sufficiently dissolved, and as a result, it may not be possible to remove the water-soluble polymer by washing. In addition, separating, through an operation such as filtration or centrifugal separation, the cellulose acylate particles not dissolved in the solvent from the solvent in which the water-soluble polymer is dissolved becomes difficult.

The mixing temperature of the dispersion and the solvent is preferably from 0°C to 200°C, more preferably from 20°C to 110°C, and even more preferably from 40°C to 80°C. At temperatures lower than 0°C, the water-soluble polymer may not be sufficiently dissolved and may be difficult to remove by washing, and at temperatures higher than 200°C, deformation, aggregation, or the like of the particles may occur, and it may be difficult to take out the particles while maintaining the desired shape of the particles.

A duration for the mixing of the dispersion and the solvent is not particularly limited and may be appropriately adjusted. For example, the duration may be not shorter than 0.5 hours, not shorter than 1 hour, not shorter than 3 hours, or not shorter than 5 hours, and may be not longer than 6 hours.

In addition, the method of mixing the dispersion and the solvent is not particularly limited as long as the water-soluble polymer can be dissolved and removed. For example, the dispersion and the solvent can be stirred using a stirring device, such as an ultrasonic homogenizer or a Three-One Motor, and thus the water-soluble polymer can be efficiently removed from the dispersion even at room temperature.

For example, when a Three-One Motor is used as the stirring device, the rotational speed during mixing the dispersion and the solvent may be, for example, from 5 rpm to 3000 rpm. This can more efficiently remove the water-soluble polymer from the dispersion. In addition, this also efficiently removes the plasticizer from the dispersion.

In the method for producing a cellulose acylate composition according to an embodiment of the present disclosure, during and/or after the third step, one or more metal compounds selected from an alkali metal compound and an alkaline earth metal compound are added to the cellulose acylate particles. As a result, with this method, the cellulose acylate composition according to the present disclosure, which contains metal compound-containing particles that act as a neutralizing agent and an antiblocking agent can be produced.

As the alkali metal compound and the alkaline earth metal compound, the compounds described above as the metal compound contained in the cellulose acylate composition can be used alone or in a combination of two or more types. Hydroxides, oxides, or carbonates of alkali metals or alkaline earth metals are preferable, and calcium carbonate, calcium hydroxide, magnesium carbonate, and magnesium hydroxide are more preferable.

The amount of the metal compound added is preferably from 0.01 parts by weight to 1.0 parts by weight per 100 parts by weight of the cellulose acylate. From the viewpoint of improving the stability over time and the tactile sensation, the addition amount of the metal compound may be 0.02 parts by weight or greater, or may be 0.05 parts by weight or greater. Furthermore, from the viewpoint that the pH value satisfies the standard, the addition amount of the metal compound may be 0.8 parts by weight or less, or may be 0.06 parts by weight or less.

Examples of a method for adding the metal compound after the third step includes a method in which the metal compound is added to the cellulose acylate particles produced in the third step, and then the materials are mixed using a mixer such as a ball mill. The metal compound may be added in powder or particulate form, or as a solution or suspension.

Examples of the method for adding the metal compound during the third step include a method in which the metal compound is added to the solvent for removing the water-soluble polymer, or a method in which a solution containing the metal compound is used in place of this solvent. In other words, in this method, by washing the dispersion described above with a solution containing the metal compound in the third step, while the metal compound is added, the water-soluble polymer is removed as well.

In the third step, in a case where the stirring and mixing of the dispersion and the solvent and the filtration are repeated a plurality of times to remove the water-soluble polymer, washing with a solution containing a metal compound is preferably implemented at least once, and washing with a solution containing a metal compound is more preferably implemented at the end of the third step. In consideration of the water-soluble polymer removal effect, the solution containing the metal compound is preferably an aqueous solution.

In a case where the metal compound is added during the third step, compared with a case in which the metal compound is added after the third step, a cellulose acylate composition including metal compound-containing particles having a smaller particle size is produced. Through this, the tactile sensation of the produced cellulose acylate composition is remarkably improved. Therefore, the method of adding the metal compound during the third step is more preferable.

Examples

The effects of the present disclosure will be clarified by the examples below, but the present disclosure should not be construed as being limited in terms of the technical scope on the basis on the description of the examples.

Example 1

100 parts by weight of cellulose diacetate (available from Daicel Corporation, total degree of substitution = 2.4) and 22 parts by weight of triacetin (available from Daicel Corporation) as a plasticizer were blended in a dry state, dried at 80°C for 12 hours or longer, and then further stirred and mixed using a Henschel mixer (available from Nippon Coke & Engineering Co., Ltd.), and a mixture of the cellulose acetate and the plasticizer was obtained. The resulting mixture was fed into a twin-screw extruder (PCM30 available from Ikegai Corp., a cylinder temperature of 200°C, a die temperature of 220°C), melt-kneaded, extruded, and pelletized, and a kneaded product was formed.

Subsequently, 34 parts by weight of the pellets of the resulting kneaded product and 66 parts by weight of polyvinyl alcohol (available from The Nippon Synthetic Chemical Industry Co., Ltd., melting point of 190°C, saponification degree of 99.1%) as a water-soluble polymer were blended in a dry state, and then fed into a twin-screw extruder (PCM30 available from Ikegai Corp., cylinder temperature of 220°C, die temperature of 220°C) and extruded, and a dispersion was formed.

The resulting dispersion was combined with pure water (a solvent) such that the concentration was not higher than 5 wt.% (weight of dispersion/(weight of dispersion + weight of pure water) x 100), and the mixture was stirred using a Three-One Motor (BL-3000 available from Shinto Scientific Co., Ltd.) at a rotational speed of 200 rpm and a temperature of 80°C for 5 hours. The solution after stirring was filtered with filter paper (No. 5A available from ADVANTEC), and the filtered product was removed. The removed filtered product was prepared again using pure water such that the concentration of the dispersion was 5 wt.% or less, the mixture was further stirred at a rotational speed of 200 rpm and temperature of 80°C for 5 hours, after which the solution was filtered, and the filtered product was removed.

Calcium hydroxide (available from Fujifilm Wako Pure Chemical Corporation) was dissolved in pure water to prepare a calcium hydroxide aqueous solution having a concentration of 1000 ppm. The calcium hydroxide aqueous solution and pure water were added to the removed filtered product. The added amounts of the pure water and the calcium hydroxide aqueous solution were 600 parts by weight and 15 parts by weight (0.015 wt.% as calcium hydroxide), respectively, per 100 parts by weight of the cellulose diacetate. The filtered product to which the pure water and calcium hydroxide aqueous solution were added was then placed in a Three-One Motor, stirred at a temperature of 80°C and a rotational speed of 200 rpm for 30 minutes, and then filtered to obtain a cellulose acylate composition of Example 1.

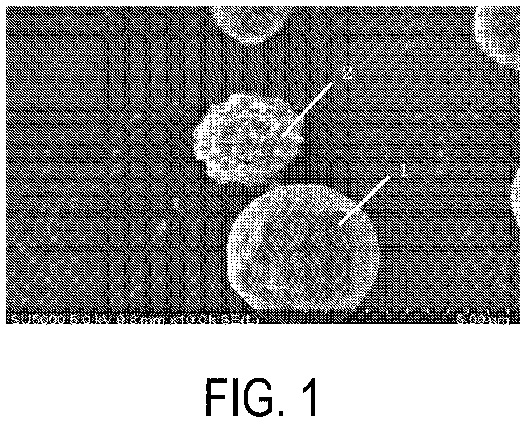

The obtained cellulose acylate composition of Example 1 was observed with an electron microscope, and the image of FIG. 1 was obtained. In FIG. 1, a cellulose acylate particle is denoted by reference numeral 1, and a metal compound-containing particle is denoted by reference numeral 2. From FIG. 1, it was confirmed that the metal compound-containing particles were aggregates and existed separately from the cellulose acylate particles. In addition, when elemental analysis was performed on the obtained cellulose acylate composition of Example 1 using an energy dispersive X-ray spectrometer (EDS), it was found that the main component of the metal compound-containing particles was calcium carbonate.

Examples 2 to 5, 8 and 10

Cellulose acylate compositions of Examples 2 to 5, 8 and 10 were obtained in the same manner as in Example 1 with the exception that the type and amount of the additive added to the filtered product were changed as shown in Tables 1 and 2 below.

Example 6

A cellulose acylate composition of Example 6 was obtained in the same manner as in Example 1 with the exception that cellulose acetate propionate (available from Eastman Chemical Company, CAP-482-0.5, degree of acetyl substitution = 0.18, degree of propionyl substitution = 2.40) was used in place of cellulose diacetate.

Example 7

A cellulose acylate composition of Example 7 was obtained in the same manner as in Example 1 with the exception that cellulose acetate butyrate (available from Eastman Chemical Company, CAB-171-15, degree of acetyl substitution = 2.40, degree of butyryl substitution = 0.71) was used in place of cellulose diacetate.

Comparative Example 1

Cellulose acylate particles of Comparative Example 1 were obtained by repeating three times an operation in which pure water was combined with the filtered product without adding the calcium hydroxide aqueous solution, and the mixture was stirred for 5 hours at a temperature of 80°C and a rotational speed of 200 rpm using a Three-One Motor and then subjected to filtration.

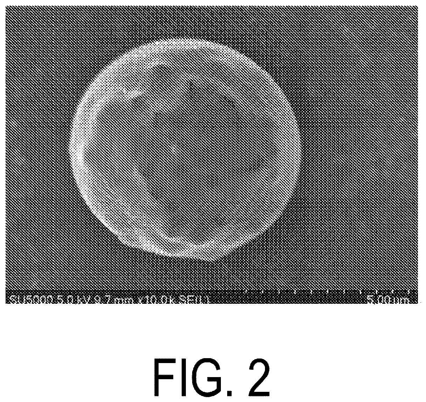

An electron micrograph of the obtained cellulose acylate particles of Comparative Example 1 is shown in FIG. 2. Through observation with the electron microscope, it was confirmed that in Comparative Example 2, metal compound-containing particles such as those denoted by reference numeral 2 in FIG. 1 were not present.

Example 9

Calcium carbonate (available from Fujifilm Wako Pure Chemical Corporation, particle size: 1 to 3 μm, average particle size: 2 μm) was added at an amount of 100 ppm to the cellulose acylate particles of Comparative Example 1, and the mixture was placed in a ball mill (available from AS ONE Corporation) and the ball mill was subjected to rotation on a pot mill rotating table (AN 3 S, available from Nitto Kagaku Kogyo Co., Ltd.) for 3 hours to homogenize the cellulose acylate particles and calcium carbonate. And thus a cellulose acylate composition of Example 9 was obtained.

[Measurement of Physical Properties and Evaluation Test]

The metal concentration, average particle size, sphericity, and degree of surface smoothness of the obtained Examples and Comparative Example were measured by the following methods, and the tactile sensation, the pH immediately after production, the pH after storage, and the odor were evaluated. The obtained results are shown in Tables 1 and 2.

[Metal Concentration]

The metal concentration in each Example and Comparative Example was measured by absorption photometry. Specifically, 3.0 g of the sample was collected in a crucible, carbonized on an electric heater, and then incinerated and converted to an ash in an electric furnace at 750 to 850°C for about 2 hours. The crucible was covered with a lid and allowed to cool, after which 25 ml of a 0.07% hydrochloric acid solution was added, and the contents were dissolved by heating on a hot plate. After cooling, the solution in the crucible was placed in a Nalgene flask having a volume 200 ml. The crucible was washed with distilled water, after which the washing liquid was also placed in the Nalgene flask, and the contents were diluted by the addition of distilled water up to the marked line. The obtained solution was used as a test solution, the absorbance was measured using an atomic absorption photometer, and the metal concentration (ppm) in the sample was determined.

[Average Particle Size]

First, each of the Examples and Comparative Example was adjusted to a concentration of around 100 ppm using pure water, and a suspension was prepared using an ultrasonic vibrating device. Subsequently, the volume-frequency particle size distribution was determined by laser diffraction ("Laser Diffraction/Scattering Particle Size Distribution Measuring Device LA-960" available from Horiba Ltd., ultrasonic treatment for 15 minutes, refractive index (1.500, medium (water; 1.333)), and the average particle size was measured. The average particle size (nm and $\mu$m) was the value of the particle size corresponding to 50% of the integrated scattering intensity in the volume-frequency particle size distribution.

[Sphericity]

Using an image of cellulose acylate particles observed by a scanning electron microscope (SEM), the major axis lengths and the minor axis lengths of 30 randomly selected particles were measured to determine the ratio (minor axis length)/(major axis length) of each particle, and the average value of the ratios (minor axis length)/(major axis length) was used as the sphericity.

[Degree of Surface Smoothness]

A scanning electron micrograph of the cellulose acylate particles of each of the Examples and the Comparative Example was captured at a magnification from 2500 to 5000 times. The captured image that was obtained was binarized using the Winroof image processor (available from Mitani Corporation). A region including the center and/or near the center of one particle was randomly selected from the binarized image. The area ratio of a portion (shade portion) corresponding to a recess of unevenness in the region was calculated, and the degree of surface smoothness (%) of one of the particles was calculated by the following formula:

Degree of surface smoothness of one particle (%) = (1 - area ratio of recesses) x 100

Area ratio of recesses = (area of recessed portions in any region)/(area of the any region)

The average value of the degree of surface smoothness of ten randomly selected particle samples (n1 to n10) was used as the degree (%) of surface smoothness. A higher numerical value of the average value of the degree of surface smoothness indicates a higher the degree of surface smoothness. Note that the region used for calculating the area ratio may be any region smaller than the particle, including the center and/or near the center of one particle. The size of the region may be 5 $\mu$m square when the particle size is 15 $\mu$m.

[Tactile Sensation]

A sensory evaluation of the tactile sensation of each Example and the Comparative Example was conducted through a panel test by 20 panelists. The panelists touched each of the compositions and comprehensively evaluated the compositions in terms of both smoothness and moist feeling on a scale with a maximum score of 5. The average score of the 20 panelists was calculated, and the tactile sensation was evaluated according to the following criteria. Good: A, somewhat good: B, normal: C, somewhat bad: D, bad: E

[pH and pH Over Time]

For each of the Examples and the Comparative Example, the pH immediately after production and the pH (pH over time) after storage for 90 days in a constant temperature bath at 60°C were measured using a pH meter (glass electrode method). Specifically, 50 ml of distilled water was weighed in a conical beaker having a volume of 100 ml, and 2.0 g of the sample was charged therein while stirring. The mixture was stirred for 30 minutes, and then subjected to pressure filtration to collect the filtrate. The filtrate was measured with a pH meter.

[Odor]

The composition of each Example and Comparative Example was stored in a constant temperature bath at 60°C for 90 days, after which 1.5 g of the sample was sampled with a medicine spoon and subjected to smell testing, and the odor was evaluated according to the 6-step odor intensity scale on the basis of the following criteria.

0: Odorless
1: Barely detectable odor (detection threshold concentration)
2: Weak odor for which odor type is recognizable (recognition threshold concentration)
3: Easily detectable odor
4: Strong odor
5: Intense odor

[Table 1]

|  | Example 1 | Example 2 | Example 3 | Example 4 | Example 5 |
|---|---|---|---|---|---|
| Cellulose acylate | CA | CA | CA | CA | CA |
| Total degree of substitution | 2.40 | 2.40 | 2.40 | 2.40 | 2.40 |
| Addition method | When washing | When washing | When washing | When washing | When washing |
| Additive | $Ca(OH)_2$ | $Ca(OH)_2$ | $Ca(OH)_2$ | $Ca(OH)_2$ | $Mg(OH)_2$ |
| Added amount (wt.%) | 0.015 | 0.020 | 0.010 | 0.200 | 0.015 |
| Metal concentration (ppm) | 82 | 123 | 56 | 1032 | 79 |
| Average particle size ($\mu$m) | 7.6 | 7.5 | 7.6 | 7.3 | 7.6 |
| Sphericity | 0.94 | 0.94 | 0.94 | 0.9 | 0.94 |
| Degree of surface smoothness (%) | 90.0 | 90.0 | 90.0 | 88.0 | 90.0 |
| Tactile sensation | A | A | A | A | A |
| pH | 6.8 | 7.2 | 6.5 | 8.2 | 6.5 |
| pH over time | 6.5 | 7 | 6.4 | 8 | 6.2 |
| Odor | 0 | 0 | 1 | 0 | 0 |

[Table 2]

| | Example 6 | Example 7 | Example 8 | Example 9 | Example 10 | Comparative Example 1 |
|---|---|---|---|---|---|---|
| Cellulose acylate | CAP | CAB | CA | CA | CA | CA |
| Total degree of substitution | 2.58 | 3.11 | 2.40 | 2.40 | 2.40 | 2.40 |
| Addition method | When washing | When washing | When washing | Mixing | When washing | - |
| Additive | $Ca(OH)_2$ | $Ca(OH)_2$ | NaOH | $CACO_3$ | $Ca(OH)_2$ | - |
| Added amount (wt.%) | 0.015 | 0.020 | 0.007 | 0.010 | 2.000 | - |
| Metal concentration (ppm) | 85 | 125 | 80 | 101 | 9855 | 5 |
| Average particle size ($\mu$m) | 8.0 | 8.1 | 7.6 | 7.5 | 6.8 | 6.8 |
| Sphericity | 0.81 | 0.85 | 0.94 | 0.93 | 0.90 | 0.95 |
| Degree of surface smoothness (%) | 75.0 | 76.0 | 90.0 | 90.0 | 87.0 | 91.0 |
| Tactile sensation | A | A | B | C | A | B |
| pH | 6.6 | 7.2 | 6.5 | 6.3 | 9.5 | 6.1 |
| pH over time | 6.4 | 6.9 | 6.2 | 6.1 | 8.9 | 4.7 |
| Odor | 1 | 1 | 0 | 0 | 0 | 3 |

As shown in Tables 1 and 2, the compositions of the Examples suppressed a decrease in pH over time better than the composition of the Comparative Example, and did not generate an odor. In addition, it was confirmed that Examples 1 to 8 and 10, in which the alkali metal compound or the alkaline earth metal compound was added during the third step, exhibited more excellent tactile sensations in comparison to Example 9 in which the metal compound was added after the third step.

Also, as shown in Tables 1 and 2, the compositions of the Examples were highly rated in comparison to the composition of the Comparative Example. In addition, a cosmetic composition excelling in tactile sensation can be obtained by blending the compositions of the examples. From these evaluation results, the superiority of the present disclosure is clear.

Industrial Applicability

The cellulose acylate composition described above can be applied to the production of various cosmetic compositions.

Reference Signs List

    1: Cellulose acylate particle
    2: Metal compound-containing particle

Claims

1.   A cellulose acylate composition comprising cellulose acylate particles and metal compound-containing particles,

    a total degree of substitution of the cellulose acylate being from 0.7 to 2.9, and
    the metal compound being one or more types of compounds selected from an alkali metal compound and an alkaline earth metal compound.

2. The cellulose acylate composition according to claim 1, wherein the metal compound is a hydroxide, an oxide, or a carbonate of an alkali metal or an alkaline earth metal.

3. The cellulose acylate composition according to claim 2, wherein the alkali metal is sodium, and the alkaline earth metal is calcium or magnesium.

4. The cellulose acylate composition according to any one of claims 1 to 3, wherein an average particle size of the metal compound-containing particles is from 20 nm to 20 μm.

5. The cellulose acylate composition according to any one of claims 1 to 4, wherein the metal compound-containing particles are aggregates.

6. The cellulose acylate composition according to any one of claims 2 to 5, wherein the cellulose acylate composition contains the alkali metal and the alkaline earth metal at a total concentration from 50 ppm to 2000 ppm.

7. The cellulose acylate composition according to any one of claims 1 to 6, wherein an average particle size of the cellulose acylate particles is from 80 nm to 100 μm.

8. The cellulose acylate composition according to any one of claims 1 to 7, wherein a sphericity of the cellulose acylate particles is from 0.7 to 1.0.

9. The cellulose acylate composition according to any one of claims 1 to 3, wherein a degree of surface smoothness of the cellulose acylate particles is from 80% to 100%.

10. The cellulose acylate composition according to any one of claims 1 to 9, wherein a substituent of the cellulose acylate is an acyl group having from 2 to 20 carbons.

11. The cellulose acylate composition according to claim 10, wherein the acyl group is selected from an acetyl group, a propionyl group, and a butyryl group.

12. The cellulose acylate composition according to any one of claims 1 to 11, wherein

   the cellulose acylate particles contain a plasticizer, and
   the content of the plasticizer is from 2 wt.% to 40 wt.% relative to the cellulose acylate particles.

13. The cellulose acylate composition according to claim 12, wherein the plasticizer is one or more types selected from the group consisting of a citrate-based plasticizer, a glycerin ester-based plasticizer, an adipate-based plasticizer, and a phthalate-based plasticizer.

14. A cosmetic composition comprising the cellulose acylate composition described in any one of claims 1 to 13.

15. A method for producing a cellulose acylate composition, the method comprising:

   mixing cellulose acylate having a total degree of substitution from 0.7 to 2.9 and a plasticizer to form a cellulose acylate impregnated with the plasticizer;
   kneading a water-soluble polymer and the cellulose acylate impregnated with the plasticizer at a temperature from 200°C to 280°C to form a dispersion containing, as a dispersoid, the cellulose acylate impregnated with the plasticizer; and
   removing the water-soluble polymer from the dispersion to form cellulose acylate particles, wherein
   during and/or after the removing the water-soluble polymer from the dispersion to form cellulose acylate particles, one or more types of metal compounds selected from an alkali metal compound and an alkaline earth metal compound are added.

16. The production method according to claim 15, wherein during the removing the water-soluble polymer from the dispersion to form cellulose acylate particles, the metal compound is added by washing the dispersion at least once with a solution containing the metal compound.

17. The production method according to claim 15 or 16, wherein the solution containing the metal compound is an aqueous solution.

18. The production method according to any one of claims 15 to 17, wherein an addition amount of the metal compound per 100 parts by weight of the cellulose acylate is from 0.01 parts by weight to 1.0 parts by weight.

# FIG. 1

FIG. 2

## INTERNATIONAL SEARCH REPORT

International application No.

**PCT/JP2022/005803**

### A. CLASSIFICATION OF SUBJECT MATTER

*C08L 1/10*(2006.01)i; *A61K 8/02*(2006.01)i; *A61K 8/19*(2006.01)i; *A61K 8/73*(2006.01)i; *C08K 3/22*(2006.01)i; *C08K 3/26*(2006.01)i; *C08K 5/10*(2006.01)i
FI: C08L1/10; A61K8/73; A61K8/19; A61K8/02; C08K3/22; C08K3/26; C08K5/10

According to International Patent Classification (IPC) or to both national classification and IPC

### B. FIELDS SEARCHED

Minimum documentation searched (classification system followed by classification symbols)

C08K3/00-13/08; C08L1/00-101/14; A61K8/02; A61K8/00-8/99; A61Q1/00-90/00

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| X | JP 2007-056160 A (FUJIFILM CORP) 08 March 2007 (2007-03-08) claims, paragraphs [0015], [0051], [0124]-[0127], examples | 1-13 |
| A | | 14-18 |
| A | WO 2019/156116 A1 (DAICEL CORPORATION) 15 August 2019 (2019-08-15) entire text | 1-18 |
| A | JP 2004-269865 A (DAICEL CHEM IND LTD) 30 September 2004 (2004-09-30) entire text | 1-18 |
| A | JP 2007-224259 A (DAICEL CHEM IND LTD) 06 September 2007 (2007-09-06) entire text | 1-18 |
| A | WO 2016/135778 A1 (DAICEL CORPORATION) 01 September 2016 (2016-09-01) entire text | 1-18 |
| A | JP 2013-166815 A (TOYO TIRE & RUBBER CO LTD) 29 August 2013 (2013-08-29) entire text | 1-18 |

☑ Further documents are listed in the continuation of Box C.    ☑ See patent family annex.

| | |
|---|---|
| \* Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **21 April 2022** | **10 May 2022** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

**INTERNATIONAL SEARCH REPORT**

International application No.

**PCT/JP2022/005803**

**C.    DOCUMENTS CONSIDERED TO BE RELEVANT**

| Category* | Citation of document, with indication, where appropriate, of the relevant passages | Relevant to claim No. |
|---|---|---|
| A | JP 2006-282923 A (ASAHI KASEI CHEMICALS CORP) 19 October 2006 (2006-10-19) entire text | 1-18 |
| A | JP 2007-231050 A (DAICEL CHEM IND LTD) 13 September 2007 (2007-09-13) entire text | 1-18 |
| A | JP 2007-056144 A (FUJIFILM CORP) 08 March 2007 (2007-03-08) entire text | 1-18 |
| A | JP 2014-503613 A (SK INNOVATION CO., LTD.) 13 February 2014 (2014-02-13) entire text | 1-18 |
| A | JP 2007-107019 A (KONICA MINOLTA HOLDINGS INC) 26 April 2007 (2007-04-26) entire text | 1-18 |
| A | JP 2002-097300 A (FUJI PHOTO FILM CO LTD) 02 April 2002 (2002-04-02) entire text | 1-18 |
| A | JP 2007-191513 A (FUJIFILM CORP) 02 August 2007 (2007-08-02) entire text | 1-18 |
| A | WO 2017/057154 A1 (NIPPON PAPER INDUSTRIES CO., LTD.) 06 April 2017 (2017-04-06) entire text | 1-18 |
| A | WO 2017/047768 A1 (OJI HOLDINGS CORP) 23 March 2017 (2017-03-23) entire text | 1-18 |
| A | JP 2004-051942 A (DAICEL CHEM IND LTD) 19 February 2004 (2004-02-19) entire text | 1-18 |
| A | US 2004/0017022 A1 (YAMADA, Tsukasa) 29 January 2004 (2004-01-29) entire text | 1-18 |

Form PCT/ISA/210 (second sheet) (January 2015)

## INTERNATIONAL SEARCH REPORT
### Information on patent family members

International application No.

**PCT/JP2022/005803**

| Patent document cited in search report | | | Publication date (day/month/year) | Patent family member(s) | | | Publication date (day/month/year) |
|---|---|---|---|---|---|---|---|
| JP | 2007-056160 | A | 08 March 2007 | (Family: none) | | | |
| WO | 2019/156116 | A1 | 15 August 2019 | US entire text | 2020/0179261 | A1 | |
| | | | | EP | 3613794 | A1 | |
| | | | | CN | 110650994 | A | |
| | | | | KR | 10-2019-0135537 | A | |
| JP | 2004-269865 | A | 30 September 2004 | (Family: none) | | | |
| JP | 2007-224259 | A | 06 September 2007 | (Family: none) | | | |
| WO | 2016/135778 | A1 | 01 September 2016 | US entire text | 2018/0037670 | A1 | |
| | | | | EP | 3263603 | A1 | |
| | | | | TW | 201630940 | A | |
| | | | | CN | 107406519 | A | |
| JP | 2013-166815 | A | 29 August 2013 | (Family: none) | | | |
| JP | 2006-282923 | A | 19 October 2006 | (Family: none) | | | |
| JP | 2007-231050 | A | 13 September 2007 | (Family: none) | | | |
| JP | 2007-056144 | A | 08 March 2007 | (Family: none) | | | |
| JP | 2014-503613 | A | 13 February 2014 | US entire text | 2013/0234083 | A1 | |
| | | | | EP | 2640770 | A2 | |
| | | | | KR | 10-2012-0053592 | A | |
| | | | | TW | 201221563 | A | |
| | | | | CN | 103261287 | A | |
| JP | 2007-107019 | A | 26 April 2007 | (Family: none) | | | |
| JP | 2002-097300 | A | 02 April 2002 | (Family: none) | | | |
| JP | 2007-191513 | A | 02 August 2007 | (Family: none) | | | |
| WO | 2017/057154 | A1 | 06 April 2017 | US entire text | 2018/0282936 | A1 | |
| | | | | EP | 3358073 | A1 | |
| | | | | CN | 108138433 | A | |
| | | | | TW | 201726779 | A | |
| WO | 2017/047768 | A1 | 23 March 2017 | US entire text | 2018/0265597 | A1 | |
| | | | | EP | 3351562 | A1 | |
| | | | | KR | 10-2018-0050739 | A | |
| | | | | CN | 108137710 | A | |
| JP | 2004-051942 | A | 19 February 2004 | US entire text | 2005/0239925 | A1 | |
| | | | | US | 2009/0170981 | A1 | |
| | | | | EP | 1512725 | A1 | |
| | | | | EP | 2431411 | A1 | |
| | | | | CN | 1668702 | A | |
| | | | | KR | 10-0973602 | B1 | |
| | | | | TW | 200307715 | A | |
| US | 2004/0017022 | A1 | 29 January 2004 | US | 2004/0124557 | A1 | |
| | | | | AU | 8622401 | A | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2016500129 T
- JP 6187653 B
- JP 2004051942 A
- JP 6609726 B
- JP H0606307 B
- WO 9204408 A

### Non-patent literature cited in the description

- **TEZUKA.** *Carbonydr. Res.,* 1995, vol. 273, 83